# EUROPEAN PATENT SPECIFICATION

(11) **EP 3 187 156 B1**
(45) Date of publication and mention of the grant of the patent: **21.11.2018**
(21) Application number: 16206705.2
(22) Date of filing: 23.12.2016
(51) Int. Cl.: A61F 5/455, A61F 6/08

(54) **DEVICE FOR SUPPORTING THE VAGINAL WALL**
VORRICHTUNG UM DIE SCHEIDENWAND ZU UNTERSTÜTZEN
DISPOSITIF DE SOUTIEN DE LA PAROI VAGINALE

(30) Priority: 30.12.2015 IT UB20159804
(43) Date of publication of application: 05.07.2017
(73) Proprietor: Rolandi, Laura Maria, 27100 Pavia (IT); Nocito, Naima, 27100 Pavia (IT)
(72) Inventor: Rolandi, Laura Maria, 27100 Pavia (IT); Nocito, Naima, 27100 Pavia (IT)
(74) Representative: De Gregori, Antonella

(56) References cited:
- WO-A1-2007/082341
- DE-C- 381 724
- DE-C- 517 849
- DE-C- 619 358
- DE-U1- 8 708 879
- US-A- 5 771 899
- US-A- 5 827 248

## Description

The present invention relates to a device for supporting the vaginal wall, particularly indicated in the case of prolapse of the bladder within the vaginal canal.

Prolapse of the bladder within the vaginal canal, also known as cystocele, occurs in the case of weakening of the vaginal wall, such weakening often being the result of obstetric trauma (childbirth) and not, aging, deficiency of innervation of the pelvic muscles, muscular dystrophies or congenital abnormalities of the pelvic bone structures. In cystocele of moderate severity, the bladder drops slightly in the vagina; in cystocele of medium severity, the bladder sinks into the vagina until it reaches the vaginal opening, while in the more advanced condition the bladder protrudes outside the vagina. Currently, the symptoms due to minor cystocele can be alleviated by proper exercise aimed at toning the muscles of the pelvic floor. However, as a treatment for a serious relaxation of the vaginal wall, invasive remedies such as surgery are mainly used today. Surgery itself involves several drawbacks, including the need to undergo anesthesia and the difficulties associated with post-operative phase, both in terms of pain and in terms of physiological function recovery.

The Applicant has found that, in addition to the intrinsic disadvantageous aspects of surgical procedures, the surgical techniques employed nowadays do not exhibit appreciable success rates, which, on the contrary, turn out to be relatively low and variable, depending on the specific defect that must be corrected (central or lateral defect). In addition, these techniques do not allow ruling out recurrence or new prolapse.

The Applicant has therefore perceived the need to conceive a device that would allow counteracting the prolapse of the bladder within the vaginal canal, making it unnecessary to intervene surgically and at the same time offering appreciable results in terms of contrast of the symptoms of mild, medium or severe cystocele.

In the light of the above, the problem underlying the present invention is therefore to conceive a device for supporting the vaginal wall that is suitable for efficiently counteracting the prolapse of the bladder within the vaginal canal. From the prior art document US 5 827 248, it was known a device for supporting the vaginal wall comprising a bell-shaped body provided with a reinforcement ring placed at the open end of the bell-shaped, the bell-shaped body being constrained to gripping means placed at closed end of the bell-shaped body.

According to a first aspect thereof, the invention therefore relates to a device for supporting the vaginal wall comprising a bell-shaped body provided with a reinforcement ring placed at the open end of the bell-shaped body, the bell-shaped body being constrained to gripping means placed at the closed end thereof, wherein, at the closed end, the bell-shaped body comprises a plurality of outflow through holes of vaginal secretions obtained in the thickness of the same.

The Applicant has observed that a very good support of the vaginal walls can be obtained through a bell-shaped device comprising, at the open end, a reinforcement ring. Such a conformation in fact allows placing the reinforcement ring within the vagina in the most suitable position to counteract a possible prolapse of the bladder, where the action exerted by the ring is supported and maintained by the resistance provided by the body itself.

Moreover, the Applicant has also realized that a device for the support of the vaginal walls must be able to be used for prolonged times, equal to at least 12 hours. This is made possible by the presence of outflow holes placed at the closed end of the bell-shaped body, which allow preventing the stagnation of vaginal secretions.

Last but not least, the Applicant has perceived the need to be able to easily place the device within the vagina and to subsequently remove it, this being ensured by the particular conformation of the body and by the presence of the gripping means.

The present invention may have at least one of the following preferred features; the latter may in particular be combined with each other as desired to meet specific application requirements.

Preferably, the reinforcement end ring has a rigidity such as to achieve a resistance to deformation of at least 10 MPa.

Preferably, the end reinforcement ring is made as a peripheral thickening of the wall of the bell-shaped body at the open end thereof.

Preferably, the thickness of the bell-shaped body is between 0.4 mm and 1 mm, more preferably equal to 0.7 mm.

Preferably, the thickness of the wall portion of the bell-shaped body defining the end reinforcement ring is comprised between 1.5 mm and 3 mm, more preferably is equal to 2 mm.

Preferably, the bell-shaped body comprises at least one additional reinforcement ring arranged at an intermediate position between the open end and the closed end of the bell-shaped body and coaxial to the same, the at least one additional reinforcement ring being adapted to increase the resistance to deformation of the bell-shaped body.

The presence of additional reinforcement rings advantageously increases the resistance to deformation and facilitates the grip for the removal.

More preferably, the at least one additional reinforcement ring consists of a peripheral thickening of the wall of the bell-shaped body, made at at least one height of the axial extension of the bell-shaped body.

Even more preferably, the thickness of the wall portion of the bell-shaped body defining the at least one additional reinforcement ring is comprised between 10 mm and 15 mm, more preferably is equal to 12 mm.

Alternatively, at least one between the end reinforcement ring and the at least one additional reinforcement ring is made as a separate element with respect to the bell-shaped body and constrained to the same in an integral manner.

According to a further variant, at least one between the end reinforcement ring and the at least one additional reinforcement ring is made as a separate element with respect to the bell-shaped body and embedded in the thickness of the same.

Preferably, the outflow holes located at the closed end of the bell-shaped body are arranged according to at least one annular development coaxial to the bell shaped body.

Preferably, the outflow holes have a diameter of between 0.6 mm and 1 mm, more preferably equal to 0.8 mm.

Preferably, the bell-shaped body comprises a plurality of second holes formed in the thickness of the same, arranged in proximity to the open end of the bell-shaped body according to at least one annular extension coaxial to the same.

The second holes advantageously allow reducing the suction effect during the extraction of the device, thus facilitating the removal thereof.

Preferably, the gripping means comprise an elongated rod which protrudes from the closed end of the bell-shaped body, externally to the same.

Preferably, the bell-shaped body is made of biocompatible material, more preferably a silicone elastomer of hardness equal to 10 MPa, such as for example the silicone elastomer referred to as silastic Q7-4750 or the elastomer dow corning C6 250 class VI.

Alternatively, the bell-shaped body is made of a thermoplastic elastomer.

Further features and advantages of the present invention will appear more clearly from the following detailed description of some preferred embodiments thereof, made with reference to the accompanying drawings.

The different features in the single configurations may be combined with one another as desired according to the description above, to make use of the advantages resulting in a specific way from a particular combination.

In such drawings,
figure 1 is a perspective view of a preferred embodiment of the device for supporting the vaginal wall according to the present invention;
figure 2 is a side elevation view of the device in figure 1;
figure 3 is a plan view of the device in figure 1;
figure 4 is a perspective sectional view of the device in figure 1.

In the following description, identical reference numerals or symbols are used for the illustration of the figures to indicate construction elements having the same function. Moreover, for clarity of illustration, some references may be not repeated in all the figures.

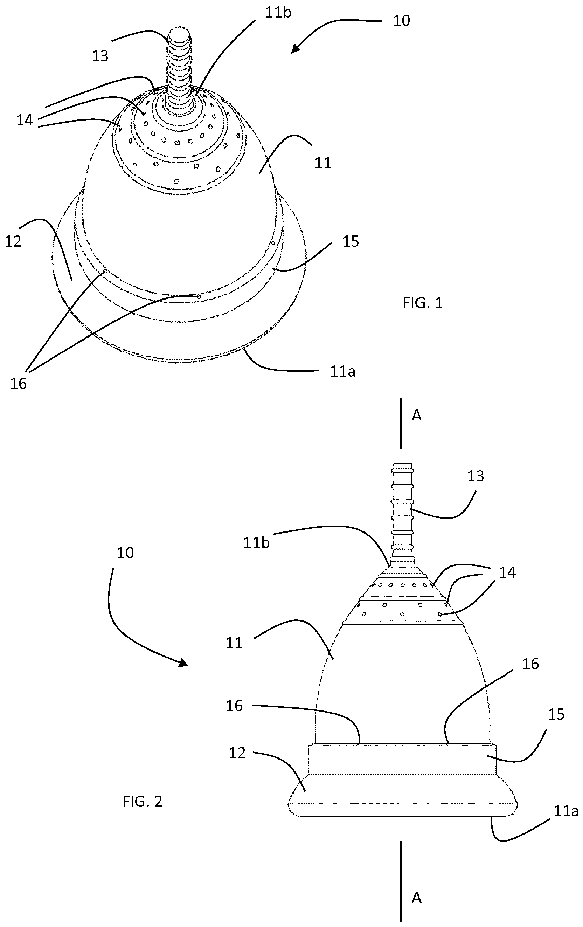

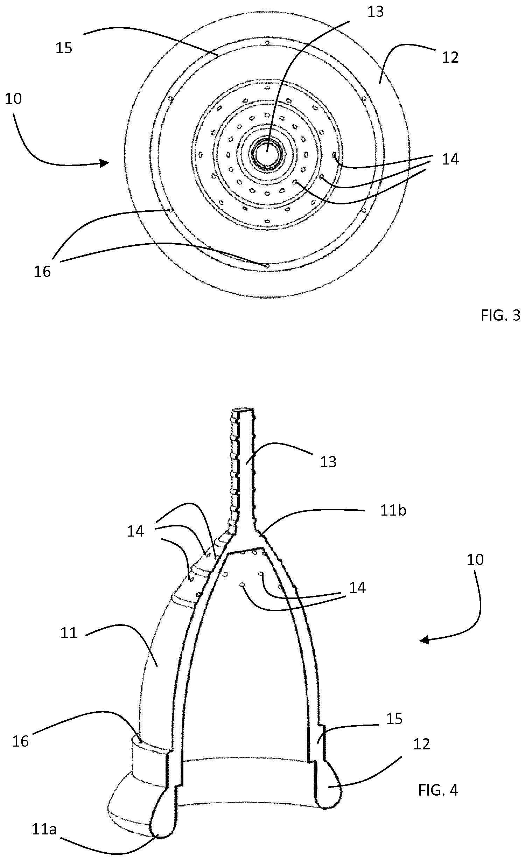

With reference to figures 1-4, reference numeral 10 globally denotes a device for supporting the vaginal wall.

The device for supporting the vaginal wall 10 comprises a bell-shaped body 11 provided with a first open end 11a and with a second closed end 11b. At the open end 11a thereof, the edge of the bell-shaped body 11 has a circular conformation.

Gripping means 13 are provided at the closed end 11b which facilitate the gripping and the axial positioning of device 10 within the vaginal canal and the subsequent removal of the same 10. In particular, the gripping means 13 of the embodiment shown are made as an elongated rod.

The edge defined by the open end 11a has an increased thickness compared to the rest of the bell-shaped body 11, which has a thickness equal to 0.7 mm. Such an edge therefore defines an end reinforcement ring 12. Specifically in the embodiment shown, the end reinforcement ring 12 has, in the point of greater thickness, a thickness of 4 mm.

Adjacent to the end reinforcement ring 12, the bell-shaped body 11 of device 10 shown in figures 1-4 has a second reinforcement ring 15, also having a thickness greater than the thickness of the walls of the bell-shaped body 11. Specifically in the embodiment shown, the additional reinforcement ring 15 has a thickness of 12 mm.

At the closed end 11b thereof, the bell-shaped body 11 comprises a plurality of outflow through holes 14 which allow the passage of vaginal secretions, thus avoiding the stagnation thereof. To this end, the outflow holes have a diameter of 0.8 mm.

The outflow holes 14 are preferably placed according to annular extension arrangements coaxial with respect to the symmetry axis A of the bell-shaped body.

The bell-shaped body 11 is also provided with second through holes 16 made in the thickness of the same 11 and arranged in proximity of the open end 10a of the bell-shaped body 11 and around the same 11a.

The operation of the device for supporting the vaginal wall according to the present invention is as follows.

Device 10 is placed within the vaginal canal with the open end 11a of the bell-shaped body 11 facing upward, preferably at the prolapse. The positioning of device 10 takes place by grasping it at the gripping means 13 which allow the manipulation thereof in an easy manner.

Once device 10 has been positioned, the end reinforcement ring 12 rests against the vaginal wall and supports it. Moreover, the resistance provided by the bell-shaped body 11 supports the action exerted by ring 12, thus maintaining the shape of ring 12 unchanged and preventing the latter from yielding under the action of the vaginal wall. Such contrasting action is also supported by the additional reinforcement ring 15 present directly beneath the end ring 12.

The presence of the outflow through holes 14 allows the vaginal secretions not to stagnate, thus allowing a prolonged use of device 10 which should otherwise be removed and cleaned frequently, thus creating discomfort to the person wearing it.

The features of the device for supporting the vaginal wall object of the present invention as well as the relevant advantages are clear from the above description.

Additional variations of the embodiments described above are possible without departing from the teaching of the invention.

Finally, it is clear that several changes and variations may be made to a device for supporting the vaginal wall thus conceived, all falling within the invention; moreover, all details can be replaced with technically equivalent elements. In the practice, the materials used as well as the sizes, can be whatever, according to the technical requirements.

## Claims

1. Device (10) for supporting the vaginal wall comprising a bell-shaped body (11) provided with a reinforcement ring (12) placed at the open end (11a) of the bell-shaped body, the bell-shaped body (11) being constrained to gripping means (13) placed at the closed end (11b) of the bell-shaped body (11), said device being **characterized in that**, at the closed end (11b), the bell-shaped body (11) comprises a plurality of outflow through holes (14) for vaginal secretions obtained in the thickness of the bell-shaped body (11).

2. Device (10) for supporting the vaginal wall according to claim 1, wherein the reinforcement end ring (12) has a rigidity such as to achieve a resistance to deformation of at least 10 MPa.

3. Device (10) for supporting the vaginal wall according to claim 1 or 2, wherein the end reinforcement ring (12) is made as a peripheral thickening of the wall of the bell-shaped body (11) at the open end (11a) thereof.

4. Device (10) for supporting the vaginal wall according to claim 3, wherein the thickness of the wall portion of the bell-shaped body defining the end reinforcement ring is comprised between 1.5 mm and 3 mm, more preferably is equal to 2 mm.

5. Device (10) for supporting the vaginal wall according to any one of the preceding claims, wherein the bell-shaped body (11) comprises at least one additional reinforcement ring (15) arranged at an intermediate height between the open end (11a) and the closed end (11b) of the bell-shaped body (11) and coaxial to the bell-shaped body (11), the at least one additional reinforcement ring (15) being adapted to increase the resistance to deformation of the bell-shaped body (11).

6. Device (10) for supporting the vaginal wall according to any one of the preceding claims, wherein the outflow holes (14) arranged at the closed end (11b) of the bell-shaped body (11) have a diameter comprised between 0.6 mm and 1 mm, more preferably equal to 0.8 mm.

7. Device (10) for supporting the vaginal wall according to any one of the preceding claims, wherein the bell-shaped body (11) comprises a plurality of second holes (16) obtained in the thickness of the same (11), the second holes (16) being arranged in proximity of the open end (11a) of the bell-shaped body (11) according to at least one annular development coaxial to the bell-shaped body (10).

8. Device (10) for supporting the vaginal wall according to claim 5, wherein the at least one additional reinforcement ring (15) is a thickening of the wall of the bell-shaped body, preferably comprised between 10 mm and 15 mm, more preferably it is equal to 12 mm.

9. Device (10) for supporting the vaginal wall according to claim 5, wherein at least one between the end reinforcement ring (12) and the at least one additional reinforcement ring (15) is a separate element with respect to the bell-shaped body (11) constrained to the same in an integral manner.

10. Device (10) for supporting the vaginal wall according to claim 5, wherein at least one between the end reinforcement ring (12) and the at least one additional reinforcement ring (15) is a separate element with respect to the bell-shaped body (11) embedded in the thickness of the same (11).

## Patentansprüche

1. Vorrichtung (10) zum Unterstützen der Scheidenwand, umfassend einen glockenförmigen Körper (11), der mit einem am offenen Ende (11a) des glockenförmigen Körpers angeordneten Verstärkungsrings (12) versehen ist, wobei der glockenförmige Körper (11) an einer Greifeinrichtung (13) festgelegt ist, die am geschlossenen Ende (11b) des glockenförmigen Körpers (11) angeordnet sind, wobei die Vorrichtung **dadurch gekennzeichnet ist, dass** der glockenförmige Körper (11) am geschlossenen Ende (11b) eine Mehrzahl von Abflussdurchgangslöchern (14) für Vaginalsekrete umfasst, die aus der Dicke des glockenförmigen Körpers (11) herausgearbeitet sind.

2. Vorrichtung (10) zum Unterstützen der Scheidenwand nach Anspruch 1, wobei der Verstärkungsendring (12) eine Steifigkeit aufweist, derart, dass ein Verformungswiderstand von wenigstens 10 MPa erzielt wird.

3. Vorrichtung (10) zum Unterstützen der Scheidenwand nach Anspruch 1 oder 2, wobei der Endverstärkungsring (12) als eine umlaufende Verdickung der Wand des glockenförmigen Körpers (11) an dem offenen Ende (11a) desselben hergestellt ist.

4. Vorrichtung (10) zum Unterstützen der Scheidenwand nach Anspruch 3, wobei die Dicke des Wandabschnitts des glockenförmigen Körpers, der den Endverstärkungsring definiert, im Bereich von 1,5 mm bis 3 mm liegt, bevorzugter gleich 2 mm ist.

5. Vorrichtung (10) zum Unterstützen der Scheidenwand nach einem der vorstehenden Ansprüche, wobei der glockenförmige Körper (11) wenigstens einen zusätzlichen Verstärkungsring (15) umfasst, der an einer Zwischenhöhe zwischen dem offenen Ende (11a) und dem geschlossenen Ende (11b) des glockenförmigen Körpers (11) und koaxial zu dem glockenförmigen Körpers (11) angeordnet ist, wobei der wenigstens eine zusätzliche Verstärkungsring (15) dazu geeignet ist, den Verformungswiderstand des glockenförmigen Körpers (11) zu erhöhen.

6. Vorrichtung (10) zum Unterstützen der Scheidenwand nach einem der vorstehenden Ansprüche, wobei die Abflusslöcher (14), die an dem geschlossenen Ende (11b) des glockenförmigen Körpers (11) angeordnet sind, einen Durchmesser im Bereich von 0,6 mm bis 1 mm, bevorzugter von 0,8 mm aufweisen.

7. Vorrichtung (10) zum Unterstützen der Scheidenwand nach einem der vorstehenden Ansprüche, wobei der glockenförmige Körper (11) eine Mehrzahl von zweiten Löchern (16) aufweist, die aus der Dicke desselben (11) herausgearbeitet sind, wobei die zweiten Löcher (16) in der Nähe des offenen Endes (11a) des glockenförmigen Körpers (11) gemäß wenigstens einer ringförmigen Abwicklung, koaxial zum glockenförmigen Körpers (10) angeordnet sind.

8. Vorrichtung (10) zum Unterstützen der Scheidenwand nach Anspruch 5, wobei der wenigstens eine zusätzliche Verstärkungsring (15) eine Verdickung der Wand des glockenförmigen Körpers ist, vorzugsweise im Bereich von 10 mm bis 15 mm, bevorzugter von 12 mm

9. Vorrichtung (10) zum Unterstützen der Scheidenwand nach Anspruch 5, wobei wenigstens entweder der Endverstärkungsring (12) oder der wenigstens eine zusätzliche Verstärkungsring (15) ein separates Element in Bezug auf dem glockenförmigen Körper (11) ist und an demselben einstückig festgelegt ist.

10. Vorrichtung (10) zum Unterstützen der Scheidenwand nach Anspruch 5, wobei wenigstens entweder der Endverstärkungsring (12) oder der wenigstens eine zusätzliche Verstärkungsring (15) ein separates Element in Bezug auf den glockenförmigen Körper (11) ist und in die Dicke desselben (11) eingebettet ist.

## Revendications

1. Dispositif (10) pour supporter la paroi vaginale comprenant un corps en forme de cloche (11) muni d'une bague de renforcement (12) placée à l'extrémité ouverte (11a) du corps en forme de cloche, le corps en forme de cloche (11) étant contraint à moyens de préhension (13) placés à l'extrémité fermée (11b) du corps en forme de cloche (11), ledit dispositif étant **caractérisé en ce que**, à l'extrémité fermée (11b), le corps en forme de cloche (11) comprend une pluralité de trous de passage d'écoulement (14) pour les sécrétions vaginales obtenus à travers l'épaisseur du corps en forme de cloche (11).

2. Dispositif (10) pour supporter la paroi vaginale selon la revendication 1, dans lequel la bague d'extrémité de renforcement (12) a une rigidité de manière à atteindre une résistance à la déformation d'au moins 10 MPa.

3. Dispositif (10) pour supporter la paroi vaginale selon la revendication 1 ou 2, dans lequel la bague d'extrémité de renforcement (12) est constituée comme un épaississement périphérique de la paroi du corps en forme de cloche (11) à son extrémité ouverte (11a).

4. Dispositif (10) pour supporter la paroi vaginale selon la revendication 3, dans lequel l'épaisseur de la partie de paroi du corps en forme de cloche définissant la bague de renforcement est comprise entre 1,5 mm et 3 mm, plus préférentiellement elle est égale à 2 mm.

5. Dispositif (10) pour supporter la paroi vaginale selon l'une quelconque des revendications précédentes, dans lequel le corps en forme de cloche (11) comprend au moins une bague de renforcement supplémentaire (15) agencée à une hauteur intermédiaire entre l'extrémité ouverte (11a) et l'extrémité fermée (11b) du corps en forme de cloche (11) et coaxiale au corps en forme de cloche (11), l'au moins une bague de renforcement supplémentaire (15) étant adaptée pour augmenter la résistance à la déformation du corps en forme de cloche (11).

6. Dispositif (10) pour supporter la paroi vaginale selon l'une quelconque des revendications précédentes, dans lequel les trous d'écoulement (14) agencés à l'extrémité fermée (11b) du corps en forme de cloche (11) ont un diamètre compris entre 0,6 mm et 1 mm, plus préférentiellement égale à 0,8 mm

7. Dispositif (10) pour supporter la paroi vaginale selon l'une quelconque des revendications précédentes, dans lequel le corps en forme de cloche (11) comprend une pluralité de seconds trous (16) obtenus dans son épaisseur (11), les seconds trous (16) étant agencés à proximité de l'extrémité ouverte (11a) du corps en forme de cloche (11) selon au moins un développement annulaire coaxial au corps en forme de cloche (10).

8. Dispositif (10) pour supporter la paroi vaginale selon la revendication 5, dans lequel l'au moins une bague de renforcement supplémentaire (15) est un épaississement de la paroi du corps en forme de cloche, préférentiellement compris entre 10 mm et 15 mm, plus préférentiellement il est égal à 12 mm.

9. Dispositif (10) pour supporter la paroi vaginale selon la revendication 5, dans lequel au moins une entre la bague d'extrémité de renforcement (12) et l'au moins une bague de renforcement supplémentaire (15) est un élément séparé par rapport au corps en forme de cloche (11) contraint au même de façon intégrée.

10. Dispositif (10) pour supporter la paroi vaginale selon la revendication 5, dans lequel au moins une entre la bague d'extrémité de renforcement (12) et l'au moins une bague de renforcement supplémentaire (15) est un élément séparé par rapport au corps en forme de cloche (11) incorporé dans l'épaisseur du même (11).
